# EUROPEAN PATENT APPLICATION

(11) **EP 4 129 392 A1**
(43) Date of publication of application: **08.02.2023**
(21) Application number: 21780968.0
(22) Date of filing: 02.04.2021
(51) Int. Cl.: A61M 37/00

(54) **FAST-DISSOLVING MICRONEEDLE**

(30) Priority: 03.04.2020 JP 2020067874
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); TANAKA, Hiroshi, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2021/014279
(87) International publication number: WO 2021/201266

(57) **Abstract**

Rapid dermal administration of a skin valuable material with a microneedle array. A microneedle array containing a low molecular weight component of a water-soluble polymer and a skin valuable material as essential components. The low molecular weight component of the water-soluble polymer preferably has a molecular weight of 100,000 or less and 2,000 or more, or an aqueous solution of 5 mass% of the low molecular weight component preferably has a viscosity (20°C) of 6 dPa●S or less.

## Description

### TECHNICAL FIELD

The present invention relates to the technical field of rapid dermal administration of skin valuable materials.

### BACKGROUND ART

Regarding dermal administration of skin valuable materials as cosmetics and quasi drugs, lotions and creams are common, and microneedle administration has also been recently reported. For example, there have been proposed a functional micropile (Patent Document 1) including, on a substrate, a pile that is formed of a carbohydrate dissolving and disappearing in vivo, such as maltose and that has a tetragonal prism shape or cylindrical shape having a length of 0.5 to 500 um and having a square or circular cross section having one side or a diameter of 0.1 to 100 um, and a needle for skin (Patent Literature 2) including a plurality of needles that contain a biodegradable material such as polylactic acid or maltose as a component and are provided around a central member.

Administration of the skin valuable material by the microneedle is generally performed for 1 hour to 1 night (approximately 8 hours), and it has been an object to administer the skin valuable material in a shorter time. The present inventors have developed a microneedle that can be dissolved within 30 minutes by containing a specific monosaccharide and/or disaccharide in an intradermally soluble microneedle having a water-soluble polymer as a base (Patent Document 3).

### PRIOR ART DOCUMENT

### PATENT DOCUMENT

Patent Document 1: JP 2003-238347 A
Patent Document 2: JP 2006-346126 A
Patent Document 3: JP 2013-189432 A

### SUMMARY OF THE INVENTION

### PROBLEMS TO BE SOLVED BY THE INVENTION

However, containing a monosaccharide and/or disaccharide leads to disadvantages such as decrease in mechanical strength of the microneedle and restrictions on the content. That is, increase in the content of the monosaccharide and/or disaccharide makes the microneedle brittle, causing inconvenience in production and use. An object of the present invention is to rapidly administer a skin valuable material to the skin by a microneedle array. When used for cosmetic purposes, the microneedle is inserted into the epidermis including the stratum corneum of the skin, and the needle tip is rapidly dissolved, so that administration can be performed in a short time. The thickness of the stratum corneum of human skin is usually 10 to 20 um. A specific object of the present invention is to provide a microneedle array in which 10 um or more of the needle tip is dissolved within 15 minutes.

### MEANS FOR SOLVING THE PROBLEM

As a result of intensive studies on the composition of the base of the microneedle array, the present inventors have paid attention to the molecular weight or characteristics of the water-soluble polymer as the base, and have found that a desired object can be achieved by using a certain amount of a component having a specific low molecular weight or a component exhibiting a specific viscosity, thereby completing the present invention.

The present invention is as follows.
[1] A microneedle array containing a low molecular weight component of a water-soluble polymer and a skin valuable material as essential components.
[2] The microneedle array according to [1], in which the low molecular weight component of the water-soluble polymer has a molecular weight of 100,000 or less and 2,000 or more, or an aqueous solution of 5 mass% of the low molecular weight component has a viscosity at 20°C of 6 dPa·S or less.
[3] The microneedle array according to [2], in which the low molecular weight component of the water-soluble polymer has a molecular weight of 50,000 or less and 2,000 or more.
[4] The microneedle array according to [2], in which the low molecular weight component of the water-soluble polymer has a molecular weight of 10,000 or less and 2,000 or more.
[5] The microneedle array according to [1], further containing a high molecular weight component of a water-soluble polymer, in which
   the high molecular weight component is a water-soluble polymer having a molecular weight of more than 100,000 or a water-soluble or water-swellable polymer component having a viscosity at 20°C of a 5 mass% aqueous solution of more than 6 dPa●S,
   the low molecular weight component of the water-soluble polymer has a molecular weight of 10,000 or less and 2,000 or more, and
   an addition amount of the high molecular weight component is 10 parts by mass or more with respect to 100 parts by mass of the microneedle array.
[6] The microneedle array according to any one of [1] to [5], containing 20 parts by mass or more of the low molecular weight component of the water-soluble polymer and 0.1 to 60 parts by mass of the skin valuable material with respect to 100 parts by mass of the microneedle array.
[7] The microneedle array according to any one of [1] to [6], in which a substrate portion of the microneedle array has a thickness of 60 um or less, and a support having a hardness of 3N or less in a flexibility test is provided on a back surface of the microneedle array.
[8] The microneedle array according to any one of [1] to [7], in which 10 um or more from a tip of a needle portion is dissolved within 15 minutes after dermal administration.
[9] The microneedle array according to any one of [1] to [8], in which the back surface of the microneedle array is fixed on an adhesive layer.
[10] The microneedle array according to any one of [1] to [9], in which the low molecular weight component of the water-soluble polymer is polyvinyl alcohol having a low degree of saponification.
[11] The microneedle array according to any one of [1] to [9], in which the low molecular weight component of the water-soluble polymer is hydroxypropyl cellulose.
[12] The microneedle array according to any one of [1] to [9], in which the low molecular weight component of the water-soluble polymer is hyaluronic acid.

### EFFECT OF THE INVENTION

According to the present invention, a skin valuable material can be administered to the skin in a short time, and the user is free from long-time application of the microneedle patch.

### BRIEF DESCRIPTION OF THE DRAWING

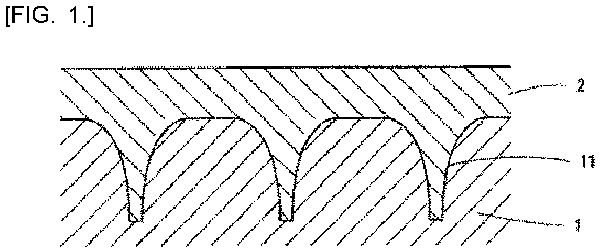

FIG. 1 is a cross-sectional view illustrating an example of a method for producing a microneedle array of the present invention.

### MODE FOR CARRYING OUT THE INVENTION

The microneedle array of the present invention contains a low molecular weight component of a water-soluble polymer and a skin valuable material. Details will be described below.

### Water-soluble polymer

Specific strategies useful in the composition and method according to the present invention are to use a low molecular weight water-soluble polymer as a main base. Here, the composition means a microneedle array as a finished product. One hundred parts by mass of the composition is equivalent to 100 parts by mass of the microneedle array. More specifically, a component having a molecular weight of 100,000 or less and 2,000 or more, or a viscosity (20°C) of a 5 mass% aqueous solution of 6 dPa●S or less was used in an amount of 20 parts by mass or more with respect to 100 parts by mass of the composition, and this was used as a main component of the base. A component having a molecular weight of more than 100,000 of the water-soluble polymer has sufficient mechanical strength, but has large entanglement of molecules, and thus takes a long time to be dissolved by moisture in the skin. In addition, a water-soluble component having a molecular weight of less than 2,000 has good solubility, but has poor mechanical strength, making the microneedle array brittle, which is inconvenient in production, storage, or use. The low molecular weight water-soluble polymer having a molecular weight of 2,000 or more and 100,000 or less has both advantageous properties in microneedle forming such as stickiness inherent in the polymer and a property of a low molecular weight water-soluble substance that dissolves in a short time. By using such a low molecular weight water-soluble polymer as a main component of the base, a microneedle that is soluble in a short time and excellent in mechanical strength has become possible. Regarding the upper limit of the molecular weight, a low molecular weight water-soluble polymer can be used as long as the mechanical strength of the microneedle is not impaired. A water-soluble polymer having a molecular weight of 50,000 or less and 2,000 or more or a molecular weight of 10,000 or less and 2,000 or more can sufficiently achieve the object of the present invention.

Examples of the low molecular weight component of the water-soluble polymer or the component having a viscosity (20°C) of a 5 mass% aqueous solution of 6 dPa●S or less include hydrolyzed sodium hyaluronate, micro sodium hyaluronate (molecular weight: 10,000 or less), hyaluronic acid oligomers and derivatives thereof, sodium chondroitin sulfate (molecular weight: 100,000 or less), hydroxypropyl cellulose (HPC, molecular weight 150,000 or less), proteoglycan (molecular weight: 100,000 or less), gelatin (molecular weight: 100,000 or less), polyvinylpyrrolidone (molecular weight: 100,000 or less), hydrolyzed collagen, carboxymethyl cellulose (molecular weight: 100,000 or less), polyethylene glycol (molecular weight: 100,000 or less), polyvinyl alcohol (molecular weight: 100,000 or less, degree of saponification: 90% or less and 50% or more), dextran (molecular weight: 100,000 or less), dextrin (molecular weight: 100,000 or less), polyacrylic acid-based polymers (molecular weight: 100,000 or less), polyacrylamide (molecular weight: 100,000 or less), polyethylene oxide (molecular weight: 100,000 or less), and fucoidan (molecular weight: 200,000 or less). The polyvinyl alcohol having a degree of saponification of 90% or less and 50% or more is a polyvinyl alcohol having a low degree of saponification. Furthermore, the degree of saponification is preferably 80% or less and 60% or more.

Examples of the high molecular weight component of the water-soluble polymer or the component having a viscosity (20°C) of a 5 mass% aqueous solution of more than 6 dPa●S include sodium hyaluronate, (molecular weight: 200,000 or more), sodium chondroitin sulfate (molecular weight: 200,000 or more), and carboxymethyl cellulose (molecular weight: 200,000 or more) hydroxypropyl cellulose (HPC, molecular weight: 200,000 or more).

On the other hand, in order to prevent the microneedle from being brittle (for the purpose of improving toughness), a water-soluble or water-swellable polymer component having a high molecular weight (water-soluble polymer having a molecular weight of more than 100,000) or a viscosity (20°C) of a 5 mass% aqueous solution of more than 6 dPa●S may be added. In that case, the addition amount of the component is desirably 30 parts by mass or less with respect to 100 parts by mass of the composition. When the low molecular weight component of the water-soluble polymer having a molecular weight of 10,000 or less and 2,000 or more is used, it is essential to add a water-soluble or water-swellable polymer component having a high molecular weight (water-soluble polymer having a molecular weight of more than 100,000) or a viscosity (20°C) of a 5 mass% aqueous solution of more than 6 dPa●S. In that case, the addition amount of the component needs to be 10 parts by mass to 30 parts by mass with respect to 100 parts by mass of the composition.

### Skin valuable material

The skin valuable material is not particularly limited as long as it is a valuable material that is absorbed through the skin. Examples thereof include a pigmentation inhibitor, a moisturizer, a metabolic activator, an antioxidant, an active oxygen scavenger/radical scavenger, a fat metabolism promoter, an anti-inflammatory agent, a blood flow promoter, a testosterone 5α reductase activity inhibitor, a hair papilla activator, and a hair growth promoter.

The content of the skin valuable material is preferably 0.1 parts by mass to 60 parts by mass with respect to 100 parts by mass of the composition.

### Pigmentation inhibitor

In the present invention, a pigmentation inhibitor can be added. Specific examples of the pigmentation inhibitor include p-aminobenzoic acid derivatives, salicylic acid derivatives, benzenesulfonamide derivatives, imidazole derivatives, naphthalene derivatives, hydroxyanthranilic acid or salts thereof and derivatives thereof, anthranilic acid derivatives, coumarin derivatives, allantoin derivatives, nicotinic acid derivatives, ascorbic acid or salts thereof and derivatives thereof, tocopherol or salts thereof and derivatives thereof, tocotrienol or salts thereof and derivatives thereof, kojic acid or derivatives thereof, oxybenzone, benzophenone, guaiazulene, shikonin, baicalin or salts thereof and derivatives thereof, baicalein or salts thereof and derivatives thereof, berberine or salts thereof and derivatives thereof, apigenin or salts thereof and derivatives thereof, luteolin or salts thereof and derivatives thereof, kaempferol or salts thereof and derivatives thereof, quercetin or salts thereof and derivatives thereof, quercitrin or salts thereof and derivatives thereof, isoquercitrin or salts thereof and derivatives thereof, rutin or salts thereof and derivatives thereof, myricetin or salts thereof and derivatives thereof, naringenin or salts thereof and derivatives thereof, hesperidin or salts thereof and derivatives thereof, glutathione or salts thereof and derivatives thereof, and ellagic acid or salts thereof and derivatives thereof.

### Moisturizer

In the present invention, a moisturizer can be added. Specific examples of the moisturizer include water-soluble polymers such as quince seed, agar or derivatives thereof, casein, glucose, galactose, mannose, xylose, fructose, maltose, isomaltose, cellobiose, gentiobiose, trehalose, pyralose, 1,3-butylene glycol, glycerin, propylene glycol, polyethylene glycol, dipropylene glycol, 1,2-pentanediol, 1,5-pentanediol, 1,2-hexanediol, 1,6-hexanediol, mannitol, and sorbitol, 1,2-propanediol, 1,3-propanediol, polypropylene glycol, 1,2-butanediol, 1,3-butanediol, 1,4-butanediol, pentylene glycol, hexylene glycol, 1,3-pentanediol, 1,4-pentanediol, erythritol, pentaerythritol, dipentaerythritol, xylitol, maltitol, inositol, panthenol or derivatives thereof, dextrin, gelatin, pectin, starch, carrageenan, carboxymethyl chitin or chitosan, chitosan salt, sulfated chitin or chitosan, phosphorylated chitin or chitosan, alginic acid or salts thereof, hyaluronic acid or salts thereof, chondroitin sulfate or salts thereof, β-1,3-glucan, β-1,4-glucan, β-1,6-glucan, glucosamine, heparin, ethyl cellulose, methyl cellulose, carboxymethyl cellulose, carboxyethyl cellulose, sodium carboxyethyl cellulose, hydroxyethyl cellulose, hydroxypropyl cellulose, nitrocellulose, crystalline cellulose, hydroxypropyl methyl cellulose, polyvinyl alcohol, polyvinyl methyl ether, polyvinylpyrrolidone, polyacrylate, carboxyvinyl polymer, dermatan sulfate, and keratan sulfate, pyrrolidonecarboxylic acid or salts thereof, polyglutamic acid or salts thereof, pyrrolidone carboxylic acid contained in natural moisturizing factors, urea, urocanic acid, betaine, sodium lactate, aspartic acid, glutamic acid, isoleucine, histidine, phenylalanine, threonine, serine, valine, proline, glycine, alanine, lysine, arginine, and ceramides such as ceramide 1, ceramide 2, ceramide 3, ceramide 4, ceramide 5, ceramide 6II, and ceramide 9.

### Metabolic activator

In the present invention, a metabolic activator can be added. Specific examples of the metabolic activator include vitamin A group: retinol or salts thereof and derivatives thereof, retinal or salts thereof and derivatives thereof, dehydroretinal or salts thereof and derivatives thereof, retinoic acid or salts thereof and derivatives thereof, carotene or salts thereof and derivatives thereof, and lycopene or salts thereof and derivatives thereof;
vitamin B group: thiamine or salts thereof and derivatives thereof, riboflavin or salts thereof and derivatives thereof, pyridoxine or salts thereof and derivatives thereof, pyridoxal or salts thereof and derivatives thereof, cyanocobalamin or salts thereof and derivatives thereof, folic acid or salts thereof and derivatives thereof, nicotinic acid or salts thereof and derivatives thereof, pantothenic acid or salts thereof and derivatives thereof, biotin or salts thereof and derivatives thereof, choline or salts thereof and derivatives thereof, and inositol or salts thereof and derivatives thereof;
vitamin C group: ascorbic acid or salts thereof and derivatives thereof;
vitamin D group: ergocalciferol or salts thereof and derivatives thereof, and cholecalciferol and salts thereof and derivatives thereof;
vitamin E group, etc.: tocopherol or salts thereof and derivatives thereof, tocotrienol or salts thereof and derivatives thereof, ubiquinone or salts thereof and derivatives thereof, linoleic acid or salts thereof and derivatives thereof, linolenic acid or salts thereof and derivatives thereof, arachidonic acid or salts thereof and derivatives thereof, carnitine or salts thereof and derivatives thereof, ferulic acid or salts thereof and derivatives thereof, and γ-oryzanol or salts thereof and derivatives thereof;
vitamin P group: rutin or salts thereof and derivatives thereof, and hesperidin or salts thereof and derivatives thereof; and
amino acids, etc.: valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, histidine or derivatives thereof, and their sulfates, phosphates, nitrates, citrates, or amino acids including amino acid derivatives such as pyrrolidonecarboxylic acid, α-hydroxy acids such as glycolic acid, citric acid, malic acid, tartaric acid, lactic acid, and succinic acid, 2-hydroxy carboxylic acids, polyhydroxycarboxylic acids or hydroxypolycarboxylic acids, lactobionic acid, photosensitizer No. 301, hinokitiol, pantothenic acid or derivatives thereof, allantoin, trimethylglycine, and proteoglycan.

### Antioxidant

In the present invention, an antioxidant can be added. Specific examples of the antioxidant include ascorbic acid or salts thereof and derivatives thereof, tocopherol or salts thereof and derivatives thereof, tocotrienol or salts thereof and derivatives thereof, butylated hydroxytoluene (BHT), butylated hydroxyanisole (BHA), coenzyme Qn (n is 7 to 10), pyrroloquinoline quinone, propyl gallate, sesamol, and carotenoids.

### Active oxygen scavenger/radical scavenger

In the present invention, an active oxygen scavenger/radical scavenger can be added. Specific examples of the active oxygen scavenger/radical scavenger include superoxide dismutase, catalase, glutathione peroxidase, bilirubin, quercetin, quercitrin, catechin, catechin derivatives, rutin or derivatives thereof, gallic acid or salts thereof and derivatives thereof, curcumin or salts thereof and derivatives thereof, transferrin, ceruloplasmin, coenzyme Qn (n is 7 to 10), uric acid, bilirubin, and metallothionein.

### Fat metabolism promoter

In the present invention, a fat metabolism promoter can be added. Specific examples of the fat metabolism promoter include xanthine derivatives (caffeine, theophylline, theobromine, xanthine, aminophylline, choline theophylline, diprophylline, proxyphylline, oxtriphylline, and the like), *Cocculus trilobus* extract, thistle extract, *Sinomenium acutum* extract, *Curcuma zedoaria* extract, *Fumaria officinalis* extract, *Platycodon grandiflorum (Platycodon, Platycodon* root) extract, *Hedera rhombea* extract, and pepper extract.

### Anti-inflammatory agent

In the present invention, an anti-inflammatory agent can be added. Specific examples of the anti-inflammatory agent include quinolinone derivatives, dibenzoxepin derivatives, thiotropocin, phthalimide derivatives, flurbiprofen, felbinac, bufexamac, suprofen, 1,4-diphenylpropylpiperazine derivatives, calxin compounds, chromanol glycosides (2-(α-D-glucopyranosyl)methyl-2,5,7,8-tetramethylchroman-6-ol), ichthammol, indomethacin, kaolin, diphenhydramine hydrochloride, d-camphor, DL-camphor, salicylic acid, sodium salicylate, methyl salicylate, acetylsalicylic acid, hydrocortisone, guaiazulene, chamazulene, chlorpheniramine maleate, diphenhydramine hydrochloride, clemastine fumarate, cyproheptadine hydrochloride, promethazine hydrochloride, piperazine derivatives, α-D-phenylglycoside derivatives, glycyrrhizic acid or salts thereof and derivatives thereof, glycyrrhetinic acid or salts thereof and derivatives thereof, mefenamic acid, phenylbutazone, ibuprofen, ketoprofen, allantoin, panthenols such as calcium pantothenate, and pantothenyl ethyl ether or salts thereof and derivatives thereof, ε-aminocaproic acid, diclofenac sodium, tranexamic acid or derivatives thereof, sulfatide, chlorpheniramine maleate, and diphenhydramine hydrochloride.

### Blood flow promoter

In the present invention, a blood flow promoter can be added. Specific examples of the blood flow promoter include tocopherol or salts thereof and derivatives thereof, tocotrienol or salts thereof and derivatives thereof, cepharanthin, carpronium chloride, eugenol derivatives, minoxidil, capsicum tincture, nonylic acid vanillylamide, cantharis tincture, ginger tincture, L-menthol, camphor, benzyl nicotinate, ichthammol, α-borneol, nonanoic acid vanillylamide, capsaicin, *Swertia japonica* extract, garlic extract, carrot extract, *Gentiana* extract, *Angelica sinensis* extract, *Zingiber officinale* (ginger) extract, and *Swertia* herb extract.

### Testosterone 5α reductase activity inhibitor, hair papilla activator, and hair growth promoter

In the present invention, a testosterone 5α reductase activity inhibitor, a hair papilla activator, and a hair growth promoter can be added. Specific examples of the testosterone 5α reductase activity inhibitor, the hair papilla activator, and the hair growth promoter include γ-amino-β-hydroxybutyrates, amine oxides, alkyl betaines, pyrimidine-N-oxide derivatives, acetylcarnitine or salts thereof, geranylgeranylacetone, hydroxamic acid derivatives or salts thereof, and proanthocyanidins.

In the present invention, in addition to the essential components described above, components usually used for external preparations for skin such as cosmetics and pharmaceuticals may be added. The components are composed of one or more components including an aqueous component, an oily component, a plant extract, an animal extract, a powder, a surfactant, an oil agent, an alcohol, a pH adjusting agent, a preservative, a thickener, a pigment, a fragrance, and the like, and these components are a part of the base, and may also serve as a valuable material due to their effect on the skin.

### Microneedle array

In the microneedle array of the present invention, Konide-shaped, pyramidal, or needle-shaped microneedles having a height of 100 to 350 um stand on a substrate having a thickness of 10 to 200 um. The skin valuable material of the microneedle is dissolved or dispersed in the base.

The method for producing the microneedle array of the present invention is not particularly limited, and the microneedle array may be produced by any conventionally known method. Examples thereof include a method involving casting a raw material solution obtained by adding other components as necessary to an aqueous solution or a suspension composed of the low molecular weight water-soluble polymer base into a mold in which the shape of the microneedle is bored, followed by drying, and then releasing the dried product from the mold. After the releasing, the released product was cut into a patch shape, and the patch is lined with an adhesive support and used.

The size of the patch (microneedle patch) including the microneedle array is 1 to 100 square cm. In the case of a size of less than 1 square cm, the effect is limited, and thus the effectiveness is hardly exhibited. In the case of a size of more than 100 square cm, a problem easily occurs in adhesion to cover the body surface. In order to cover a wide body surface, a plurality of microneedle patches having a size of 100 square cm or less may be used.

In order to stably apply the microneedle patch to the skin and retain the microneedle patch on the skin, it is not essential but more preferable that an adhesive support is provided on the back surface of the patch. Here, the term "back surface" refers to a surface opposite to a surface on which needles of the microneedle stand. The adhesive support is a support film having an adhesive layer on one side thereof.

As the adhesive layer in the present invention, an adhesive sheet made of a commercially available adhesive can be used. A rubber-based adhesive, a silicone-based adhesive, or the like can be used, and an acrylic adhesive is preferable in consideration of close adhesion to the skin and adhesion to the support film. Specific examples of the acrylic adhesive include copolymers of an alkyl acrylate and copolymers mainly composed of an alkyl acrylate with acrylic acid, acrylamide, vinyl acetate, or the like. In order to improve the tackiness to the skin, a plasticizer such as isopropyl myristate or isopropyl palmitate may be added to these adhesives. The thickness of the adhesive layer is desirably 10 um or more and 300 um or less. Specifically, a HiPAS adhesive (acrylic ester, manufactured by CosMED Pharmaceutical Co. Ltd) and a MASCOS10 adhesive (acrylic ester, manufactured by CosMED Pharmaceutical Co. Ltd) can be suitably used.

Many acrylic ester-based resins are commercially available, and in order to select resins having physical properties that can be used as an adhesive, their mechanical properties were measured.

### "Hardness measurement method"

Sheets having a thickness of 3 mm are prepared from several types of acrylic resins, and a stainless steel cylinder having a diameter of 1.5 mm is compressed from the upper surface of each of the sheets using a small desktop tester EZ Test EZSX (manufactured by Shimadzu Corporation) (compression speed: 0.5 mm/min). A compressive stress (unit N) at a depth compression of 0.1 mm from 0.1 to 0.2 mm at which a stable compressive stress is obtained in the obtained compressive stress-compression depth curve is obtained. The value obtained by the present method is defined as "hardness" in the present invention.

As a result of evaluating the hardness and the tackiness of many resins tested, the inventors found that an acrylic resin having a hardness of 3 or less is preferable as the acrylic adhesive in the present invention. Vinysol 1087FT (acrylates copolymer ammonium, manufactured by Daido Chemical Corporation), Yodosol GH800F (alkyl acrylate copolymer ammonium, manufactured by Akzo Nobel N.V.) and the like can be suitably used.

As the support film, a film made of a synthetic polymer is preferable, and the support film is required to be excellent in adhesiveness to the acrylic adhesive, be capable of maintaining strength, and be easily formed into a thin film. Specifically, the synthetic polymer is selected from polyethylene, polyethylene terephthalate (PET), polyvinylpyrrolidone, polyvinyl alcohol, and an acrylic resin. The hardness of the acrylic resin is preferably 3N or more. The value of hardness is more preferably 4 or more and 50 or less. When the hardness is less than 3, tackiness may occur in the support film, which is inconvenient for use. A non-woven fabric, a knitted fabric, or the like can also be a preferred support.

### EXAMPLES

Hereinafter, the present invention will be described in more detail by the following Examples. These Examples are merely examples for specifically describing the present invention, and the scope of the present invention is not limited to these Examples.

### Example 1

### Preparation of microneedle array

FIG. 1 is a cross-sectional view illustrating an example of a method for producing a microneedle array of the present invention. In the drawing, reference numeral 1 denotes a mold including concave portions 11 for forming Konide-shaped microneedles formed by forming a Konide-shaped microneedle pattern by a lithography method of irradiating a photosensitive resin with light and then transferring the Konide-shaped microneedle pattern through electro-casting. Reference numeral 2 denotes a microneedle raw material solution that is cast into the concave portions 11 for forming microneedles.

The concave portions 11 for forming microneedles each have a Konide shape having a base diameter of 0.6 mm, a tip diameter of 0.02 mm, and a depth of 0.15 mm, and are arranged in a lattice pattern at intervals of 0.8 mm.

An aqueous solution (microneedle raw material solution) obtained by dissolving 75 parts by mass of hydroxypropyl cellulose (manufactured by Nippon Soda Co., Ltd., trade name: NISSO HPC SSL), 20 parts by mass of hyaluronic acid (trade name "FCH-SU" manufactured by Kikkoman Biochemifa Company, molecular weight 100,000, viscosity represented by a 5 mass% aqueous solution is 4.3 dPa●S (20°C)), and 5 parts by mass of 3-o-ethylascorbic acid (Nippon Fine Chemical Co., Ltd.) in 100 parts by mass of water at room temperature was cast into the mold 1, and heated to evaporate the moisture of the aqueous solution layer. Then, the dried product was released from the mold 1, and punched into an elliptical shape (10 × 50 mm, short diameter x long diameter).

### Examples 2 to 12

Aqueous solutions (microneedle raw material solutions) having the compositions listed in Table 1 were prepared, and microneedle arrays were produced according to the production method of Example 1.

### Comparative Examples 1 to 3

Aqueous solutions (microneedle raw material solutions) having the compositions listed in Table 1 were prepared, and microneedle arrays were produced according to the production method of Example 1.

### Preparation of microneedle array with protective adhesive tape

An elliptical microneedle array (10 × 50 mm, short diameter x long diameter) was set in the central portion of a rectangular (16 x 60 mm) adhesive tape with a support having rounded corners to obtain a microneedle array with a protective adhesive tape of the present invention.

**[Table 1]**

| Examples Comparative Examples | Low molecular weight component of water-soluble polymer (part by mass) | Skin valuable material 1 (part by mass) | Skin valuable material 2 (part by mass) | High molecular weight component of water-soluble polymer (part by mass) |
|---|---|---|---|---|
| Example 1 | Sodium hyaluronate MW 100,000 (20) HPC-SSL MW 40,000 (75) | 3-O-ethylascorbic acid (5) | - | - |
| Example 2 | Sodium hyaluronate MW 100,000 (50) | Retinol (0.1) | Glucose (30) | Sodium hyaluronate MW 2,000,000 (20) |
| Example 3 | Sodium hyaluronate MW 50,000 (30) | Taurine (0.5) | Trehalose (39.5) | - |
| | HPC-SL MW 100,000 (30) | | | |
| Example 4 | HPC-SL MW 100,000 (53) | L-ascorbic acid 2-glucoside (2) | Glycerin (5) | Sodium hyaluronate MW 2,000,000 (20) |
| | Dextran MW 70,000 (20) | | | |
| Example 5 | Sodium hyaluronate MW 100,000 (50) | Proline (1) | - | - |
| | HPC-SL MW 100,000 (49) | | | |
| Example 6 | Sodium hyaluronate MW 100,000 (50) | Horse placenta extract (5) | Glucosamine (10) | - |
| | HPC-SL MW 100,000 (25) | | | |
| | Hydrolyzed collagen MW 80,000 (10) | | | |
| Example 7 | HPC-SL MW 100,000 (50) | Sodium hyaluronate MW 2,000 (7) | Proline (3) | HPC-H MW 1,000,000 (30) |
| | Hydrolyzed collagen MW 80,000 (10) | | | |
| Example 8 | HPC-SL MW 100,000 (60) | - | BG (3.9) | Sodium hyaluronate MW 2,000,000 (20) (also valuable material) |
| | HPC-SSL MW 40,000 (16) | | | |
| Example 9 | HPC-SL MW 100,000 (20) | Urea (5) | - | Sodium hyaluronate MW 600,000 (75) |
| Comparative Example 1 | - | Urea (5) | - | Sodium hyaluronate MW 2,000,000 (95) |
| Comparative Example 2 | - | Proline (10) | - | HPC-H MW 1,000,000 (90) |
| Comparative Example 3 | - | Glycerin (5) | - | Sodium hyaluronate MW 800,000 (95) |
| Example 10 | HPC-SSL MW 40,000 (70 parts) | Retinoic acid (0.1 parts) | | Carboxymethyl cellulose MW 400,000 (29.9 parts) |
| Example 11 | Micro hyaluronic acid | | | Sodium hyaluronate MW 600,000 (40 parts) |
| | MW 5,000 or less (60 parts) | | | |
| Example 12 | Polyvinyl alcohol with low degree of saponification MW 9,000 (40 parts) | Retinal (0.1 parts) | | Sodium hyaluronate MW 1,000,000 (59.9 parts) |

**[Table 2]**

| | Solubility |
|---|---|
| | State of dissolution of needle portion 15 minutes after dermal administration is observed |
| | 20 µm or more from tip is dissolved: |
| | Excellent |
| | 10 µm or more from tip is dissolved: Good |
| | 10 µm or less from tip is dissolved: Poor |
| Example 1 | Excellent |
| Example 2 | Good |
| Example 3 | Excellent |
| Example 4 | Good |
| Example 5 | Excellent |
| Example 6 | Excellent |
| Example 7 | Good |
| Example 8 | Good |
| Example 9 | Good |
| Comparative Example 1 | Poor |
| Comparative Example 2 | Poor |
| Comparative Example 3 | Poor |
| Example 10 | Excellent |
| Example 11 | Excellent |
| Example 12 | Excellent |

In all of the microneedle arrays of Examples, 10 micrometers or more from the tip were dissolved within 15 minutes. Since the microneedle arrays of Comparative Examples contained no low molecular weight component of the water-soluble polymer, the solubility at the tip was lower than those of the products of Examples.

### DESCRIPTION OF REFERENCE SYMBOLS

- 1: Mold
- 2: Microneedle raw material solution
- 11: Concave portion for forming microneedle

## Claims

1. A microneedle array comprising a low molecular weight component of a water-soluble polymer and a skin valuable material as essential components.

2. The microneedle array according to claim 1, wherein the low molecular weight component of the water-soluble polymer has a molecular weight of 100,000 or less and 2,000 or more, or an aqueous solution of 5 mass% of the low molecular weight component has a viscosity at 20°C of 6 dPa●S or less.

3. The microneedle array according to claim 2, wherein the low molecular weight component of the water-soluble polymer has a molecular weight of 50,000 or less and 2,000 or more.

4. The microneedle array according to claim 2, wherein the low molecular weight component of the water-soluble polymer has a molecular weight of 10,000 or less and 2,000 or more.

5. The microneedle array according to claim 1, further comprising a high molecular weight component of a water-soluble polymer, wherein
the high molecular weight component is a water-soluble polymer having a molecular weight of more than 100,000 or a water-soluble or water-swellable polymer component having a viscosity at 20°C of a 5 mass% aqueous solution of more than 6 dPa●S,
the low molecular weight component of the water-soluble polymer has a molecular weight of 10,000 or less and 2,000 or more, and
an addition amount of the high molecular weight component is 10 parts by mass or more with respect to 100 parts by mass of the microneedle array.

6. The microneedle array according to any one of claims 1 to 5, comprising 20 parts by mass or more of the low molecular weight component of the water-soluble polymer and 0.1 to 60 parts by mass of the skin valuable material with respect to 100 parts by mass of the microneedle array.

7. The microneedle array according to any one of claims 1 to 6, wherein a substrate portion of the microneedle array has a thickness of 60 um or less, and a support having a hardness of 3N or less in a flexibility test is provided on a back surface of the microneedle array.

8. The microneedle array according to any one of claims 1 to 7, wherein 10 um or more from a tip of a needle portion is dissolved within 15 minutes after dermal administration.

9. The microneedle array according to any one of claims 1 to 8, wherein the back surface of the microneedle array is fixed on an adhesive layer.

10. The microneedle array according to any one of claims 1 to 9, wherein the low molecular weight component of the water-soluble polymer is polyvinyl alcohol having a low degree of saponification.

11. The microneedle array according to any one of claims 1 to 9, wherein the low molecular weight component of the water-soluble polymer is hydroxypropyl cellulose.

12. The microneedle array according to any one of claims 1 to 9, wherein the low molecular weight component of the water-soluble polymer is hyaluronic acid.
